Europäisches Patentamt

European Patent Office

Office européen des brevets

(11) Publication number: **0 247 449**
**A1**

# EUROPEAN PATENT APPLICATION

(21) Application number: **87107003.3**

(51) Int. Cl.⁴: **A61N 5/10** , A61N 5/01

(22) Date of filing: **14.05.87**

(30) Priority: **19.05.86 GB 8612128**

(43) Date of publication of application:
**02.12.87 Bulletin 87/49**

(84) Designated Contracting States:
**AT BE CH DE ES FR GB GR IT LI LU NL SE**

(71) Applicant: **VARIAN-TEM LIMITED**
**Gatwick Road**
**Crawley Sussex RH10 2RG(GB)**

(72) Inventor: **Myles, Jeremy Ronald**
**Poynters Effingham Road**
**Copthorne, Sussex(GB)**

(74) Representative: **Meddle, Alan Leonard et al**
**FORRESTER & BOEHMERT**
**Widenmayerstrasse 4/I**
**D-8000 München 22(DE)**

(54) **Improvements in or relating to a radiation therapy simulator.**

(57) A radiation therapy simulator apparatus, comprising means (1) to support a patient, a radiation source (4), means (3) to selectively position the radiation source relative to the means to support a patient, and means to control movement of the radiation source, the radiation source incorporating a collimator including a plurality of elements movable to selected positions to selectively collimate the x-ray beam from the radiation source, the apparatus further comprising a display device, which display device incorporates a representation of the collimator indicting the relative position of the collimator components at any instant.

FIG 1

## Improvements in or relating to a radiation therapy simulator

THE PRESENT INVENTION relates to a radiation therapy simulator such as is used to simulate a therapy treatment on a patient before the treatment itself is carried out.

According to one aspect of this invention there is provided a radiation therapy simulator apparatus, said radiation therapy simulator apparatus comprising means to support a patient, a radiation source, means to selectively position the radiation source relative to the means to support a patient, and means to control movement of the radiation source, the radiation source incorporating a collimator including a plurality of elements movable to selected positions to selectively collimate the x-ray beam from the radiation source, the apparatus further comprising a display device, which display device incorporates a representation of the collimator, indicating the relative position of the collimator components at any instant.

Preferably the collimator includes two pairs of collimator blades movable in orthogonal directions and two pairs of cross wires, the pairs of cross wires being parallel to each other, and being parallel to the directions of movement of the collimator blades, the collimator wires themselves being movable orthogonally, the display device displaying the position of the said blades and the said wires.

Conveniently each component of the collimator is movable independently of the remaining components.

Advantageously the representation of the collimator identifies at least some of the components constituting the collimator.

Preferably the said display is associated with a control console which may be used to control movements of the apparatus.

According to another aspect of this invention there is provided a radiation therapy simulator apparatus, said simulator apparatus comprising a table adapted to support a patient, a radiation source which is movable relative to a patient supported on the table, means to move the radiation source, and micro processor or computer means adapted to control the means which move the radiation source relative to the patient, said microprocessor or computer means being associated with a memory which retains data relating to a predetermined set of operational positions of the apparatus so that one of the stored operational conditions of the apparatus may be established by retrieving the stored data from the memory.

Preferably the radiation source includes an adjustable collimator, and data relating to various positions of the collimator is present in the retained data.

Conveniently a control console is associated with the microprocessor or computer, and a single control element may be operated on the control console to call up from the memory the data relating to a preselected condition of the apparatus.

Preferably the radiation source is a source of x-rays.

In order that the invention may be more readily understood, and so that further features thereof may be appreciated, the invention will now be described, by way of example, with reference to the accompanying drawings in which:

FIGURE 1 is a perspective view of a radiation therapy treatment simulator apparatus in accordance with the invention;

FIGURE 2 is a view of a pendant control device for use with the apparatus shown in Figure 1;

FIGURE 3 is a view of a control console for use with the apparatus of Figure 1;

FIGURE 4 is a block diagram showing the control system for the simulator shown in Figure 1;

FIGURE 5 is a plan view of a collimator arrangement present in the x-ray source of the simulator of Figure 1;

FIGURE 6 is a perspective view of the collimator arrangement shown in Figure 5;

FIGURE 7 is a view of a typical display on the display device associated with the console shown in Figure 3, and

FIGURE 8 is a view corresponding to Figure 7 showing a different display.

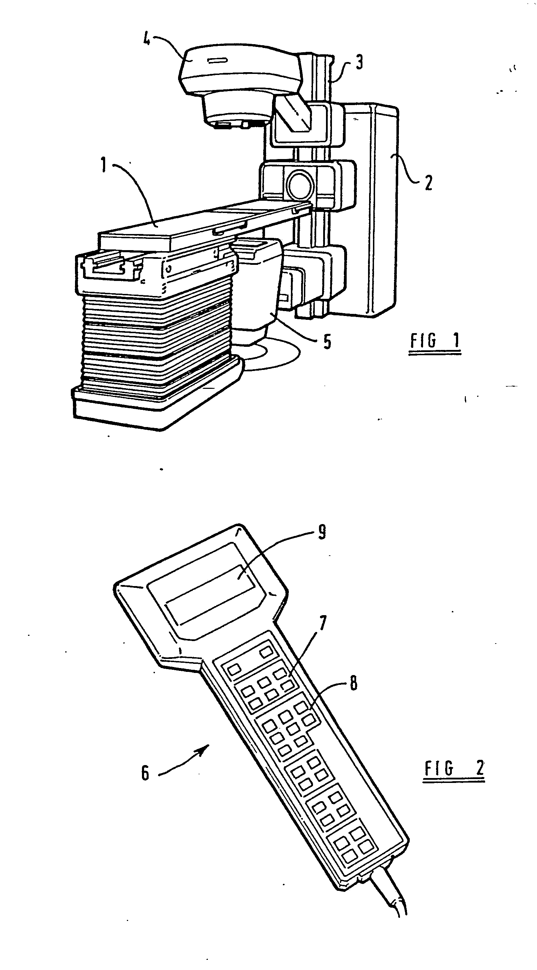

Referring initially to Figure 1 a radiation therapy simulator consists of an adjustable table 1, on which a patient may lie. The height of the table may be adjusted, and the rotational position of the table relative to the rest of the apparatus may be adjusted.

The apparatus also comprises a housing 2 which contains a computer and various control components. Movably mounted on a mast 3 present on the front of the housing 2 is a radiation source 4 which may conveniently comprise a source of x-rays. A radiation detector 5, in this embodiment a cesium iodide image intensifier, is located under the table.

The radiation source is mounted on the mast 3 in such a way that the source may be moved to many different positions to direct radiation towards a patient lying on the table 1 in an appropriately selected manner.

When the radiation therapy simulator is utilised a patient is located on the table and the x-ray source is moved through a selected sequence of movements, with radiation being directed towards the patient. An image of the part of the patient being irradiated is obtained from the radiation detector 5. The apparatus can thus be utilised to simulate a radiation therapy treatment, and the images collected from the image detector 5 will determine precisely where the radiation passed through the body of the patient. The information gathered during this exercise can subsequently be utilised in the planning and completion of a proper radiation therapy session, in which the patient is irradiated with radiation intended to destroy, for example, a tumour or the like.

As will be appreciated, the apparatus illustrated in Figure 1 is very versatile, and there are many different components present in the apparatus that can be selectively adjusted to have a desired position. To assist the operator of the apparatus a control pendant 6 is provided which is illustrated in Figure 2. The pendant has various finger operated controls 7 which can be utilised to adjust the position of the couch, the x-ray source, etc and various other controls 8 may be utilised to adjust a collimator present in the x-ray source which will be described in greater detail hereinafter. The pendant 6 may also include a back-lighted liquid crystal display 9 which can provide instantaneous numerical data relating to the positioning of various components of the apparatus.

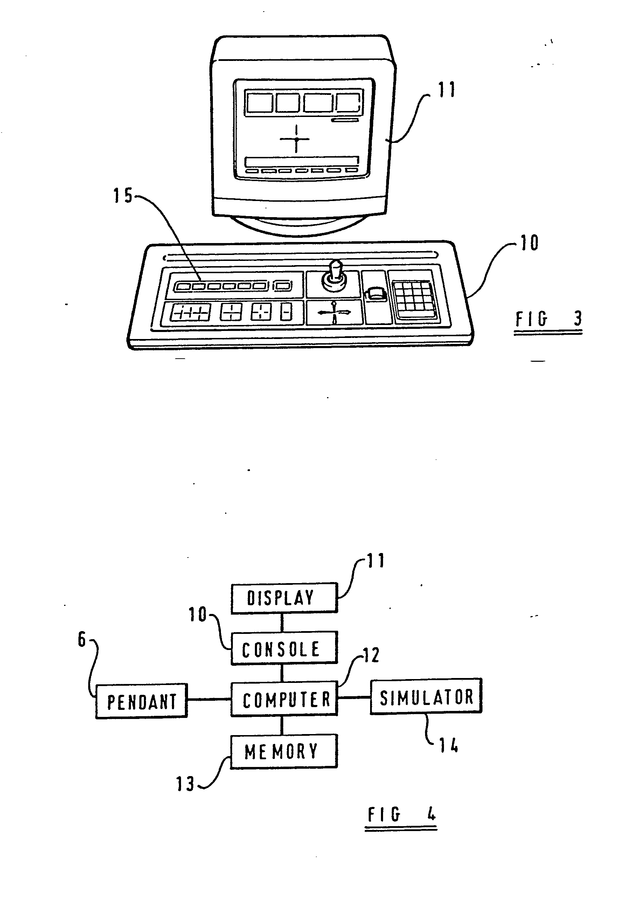

The apparatus is also provided with a remote control console as shown in Figure 3 which consists of a main console keyboard 10 and a visual display unit 11. The control console is provided with various operating buttons and a joy stick.

As can be appreciated from Figure 3 the console 10 and the associated display 11 are connected directly to a computer 12, and the pendant 6 is also directly connected to the computer. The computer is associated with a memory 13. The computer is connected to control the various operational parameters of the simulator, which is - schematically identified as box 14.

It will be appreciated that when a patient is to utilise the simulator the various components of the simulator will have to be positioned in very different positions in dependence upon the particular therapy that is to be simulated. Thus the various components of the simulator will be in one initial position when a brain tumour therapy is being simulated, and in a very different position if a stomach tumour therapy is being simulated. In order to assist the operator in dealing with patients suffering from very different complaints in an easy and straightforward manner, the described apparatus has a facility for storing, in the memory 13, various pre-selected combinations of operational parameters. Each particular group of operational parameters are associated with a single control element of a group of control elements 15 present on the control console keyboard 10. Thus it is possible for the operator to select the appropriate control 15 in accordance with the particular therapy to be simulated, and the apparatus will then immediately be adjusted, by the computer, so that all the operational parameters of the apparatus are correctly set. Thus all the movable components of the apparatus are automatically moved to an appropriate initial position. This is a major time saving feature.

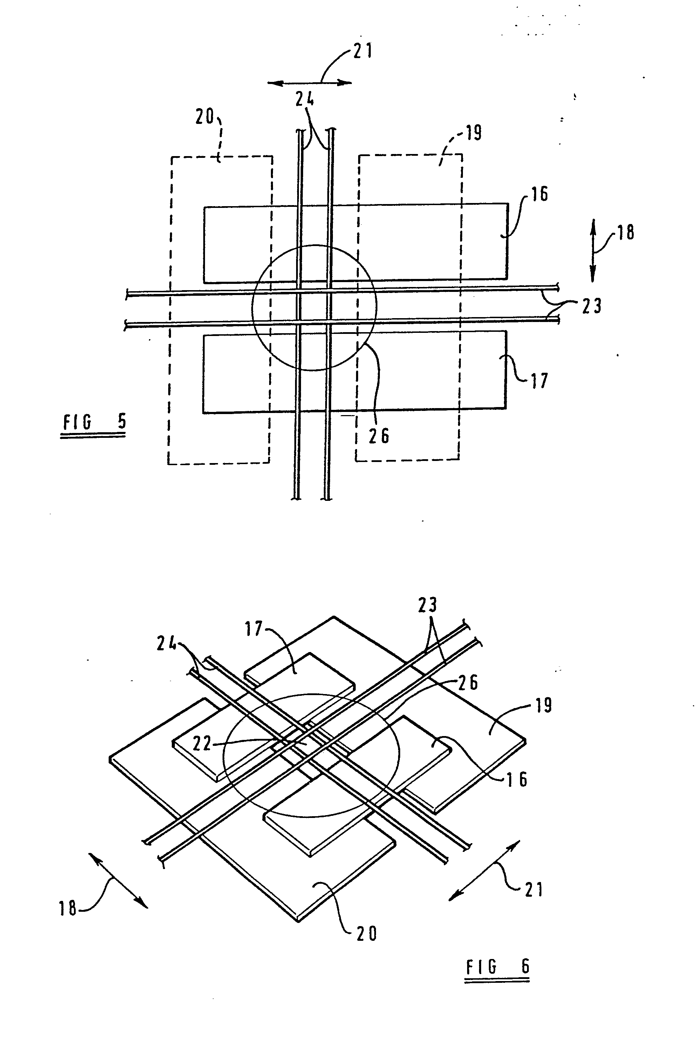

As has been mentioned previously, the x-ray source is provided with a collimator which is illustrated schematically in Figures 5 and 6. The collimator consists of a first pair of collimator blades 16, 17 which can move towards and away from each other in the direction illustrated generally by the arrow 18. The collimator blades have opposed edges which are linear and parallel, so the collimator blades can be moved until they touch each other, thus completely closing off the x-ray source. Two further collimator blades 19, 20 are provided, which are mounted transversely to the blades 16, 17. These blades also have opposing linear edges, and the blades can move towards and away from each other in the direction indicated by the arrow 21. Thus again these blades can be moved until they touch each other, thus closing off the x-ray source. In the normal mode of operation of the device the blades are separated thus defining between them a square or rectangular aperture 22. The precise configuration and orientation of this aperture may be adjusted by selectively moving the various collimator blades mentioned above, and the apparatus is provided with appropriate controls to enable this to be done. In addition the collimator assembly comprises two pairs of cross wires 23, 24, the wires 23 being parallel to the linear edges of the collimator blades 16, 17 and the wires 24 being parallel with the linear edges of the collimator blades 19, 20. The individual collimator wires may be moved, the collimator wires 23 being movable in the direction indicated by the arrow 18 and the collimator wires 24 being movable in the direction indicated by the arrow 21. Usually the collimator wires are positioned to intersect within the region of the aperture 22.

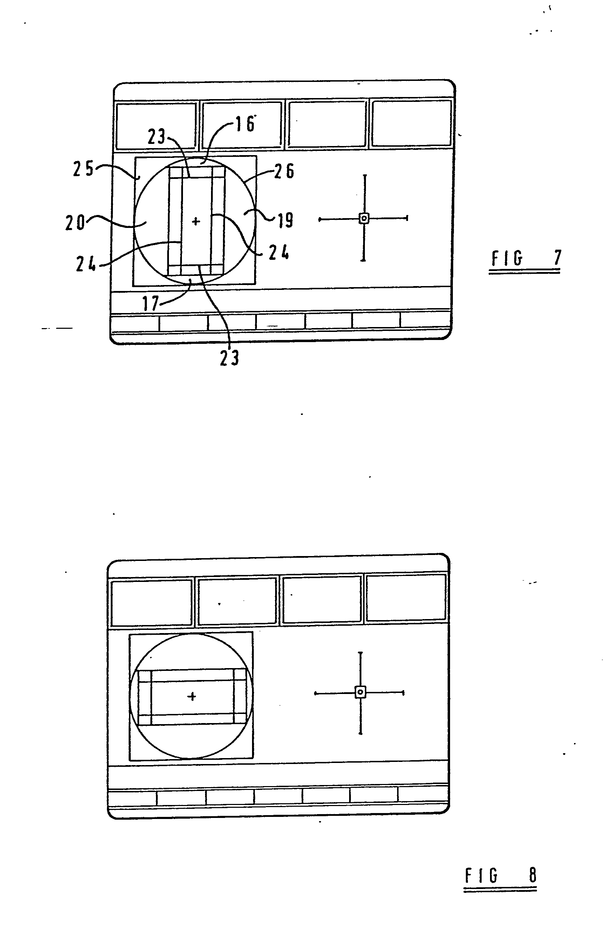

It will be appreciated that operation of the collimator is relatively complex, since there are eight movable elements present within the collimator. It is thus difficult for the operator of the apparatus to keep in mind the precise condition of the collimator. To assist the operator in this regard in the present invention the display device 11 associated with the control console 10 is provided with a representation 25 which, as can be seen from Figure 7, shows, at any instant, the precise condition of the collimator with reference to a guide circle 26 which corresponds to the notional dimension 26 of the x-ray beam as shown in Figures 5 and 6.

As can be seen from Figure 7 the area blanked off by the various collimator blades is shown, as is the position of the wires 23, 24. Figure 8 corresponds to Figure 7 and shows the various illustrated components in a different position.

It will be appreciated that the operator of the described device can readily observe, directly from the display 11 associated with the control console 10, the precise position of all the components of the collimator assembly, thus facilitating speedy and accurate operation of the collimator assembly.

If the x-ray source is rotated about its axis, so the representation of the collimator will be similarly rotated.

It is to be understood that the description of the collimator assembly given above is somewhat simplified, since the actual construction of the collimator is relatively complex. The position of various components present in the collimator assembly are sensed by means of potentiometers or the like, and relevant signals are fed to the computer, which is programmed to process the signals appropriately.

The representation of the collimator wires on the display may identify the wires of the various pairs by means of appropriate symbols to facilitate an immediate recognition of precisely which wire is which.

The features as disclosed in the foregoing description, in the claims and/or in the accompanying drawings may, both seaprately and in any combination thereof, be material for realising the invention in diverse forms thereof.

## Claims

1. A radiation therapy simulator apparatus, said radiation therapy simulator apparatus comprising means to support a patient, a radiation source, means to selectively position the radiation source relative to the means to support a patient, and means to control movement of the radiation source, the radiation source incorporating a collimator including a plurality of elements movable to selected positions to selectively collimate the x-ray beam from the radiation source, the apparatus further comprising a display device, which display device incorporates a representation of the collimator, indicting the relative position of the collimator components at any instant.

2. An apparatus according to claim 1 wherein the collimator includes two pairs of collimator blades movable in orthogonal directions and two pairs of cross wires, the pairs of cross wires being parallel to each other, and being parallel to the directions of movement of the collimator blades, the collimator wires themselves being movable orthogonally, the display device displaying the position of the said blades and the said wires.

3. An apparatus according to claim 2 wherein each component of the collimator is movable independently of the remaining components.

4. An apparatus according to claim 1, 2 or 3 wherein the representation of the collimator identifies at least some of the components constituting the collimator.

5. An apparatus according to any one of the preceding claims wherein the said display is associated with a control console which may be used to control movements of the apparatus.

6. A radiation therapy simulator apparatus, said simulator apparatus comprising a table adapted to support a patient, a radiation source which is movable relative to a patient supported on the table, means to move the radiation source, and micro processor or computer means adapted to control the means which move the radiation source relative to the patient, said microprocessor or computer means being associated with a memory which retains data relating to a predetermined set of operational positions of the apparatus so that one of the stored operational conditions of the apparatus may be established by retrieving the stored data from the memory.

7. An apparatus according to claim 6 wherein the radiation source includes an adjustable collimator, and data relating to various positions of the collimator is present in the retained data.

8. An apparatus according to claim 7 or 8 wherein a control console is associated with the microprocessor or computer, and a single control element may be operated on the control console to call up from the memory the data relating to a preselected condition of the apparatus.

9. An apparatus according to any one of the preceding claims wherein the radiation source is a source of x-rays.

10. An apparatus substantially as herein described with reference to and as shown in the accompanying drawings.

FIG 1

FIG 2

FIG 3

FIG 4

FIG 5

FIG 6

FIG 7

FIG 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | MEDICAL PHYSICS, vol. 5, no. 5, September/October 1978, pages 426-429, Am. Assoc. Phys. Med., New York, US; P.K. KIJEWSKI et al.: "Wedge-shaped dose distributions by computer-controlled collimator motion" * Pages 426,427 * | 1-8,10 | A 61 N 5/10 A 61 N 5/01 |
| X | BROWN BOVERI REVIEW, vol. 70, no. 9/10, September/October 1983, pages 312-323, Baden, CH; H. OVERHAUS: "BBC-radiotherapy systems: modular, system-oriented, comprehensive" * Page 313, right-hand column - page 314, left-hand column; pages 315-317; figures 1-3,5 * | 1,6-9, 10 | |
| A | BROWN BOVERI REVIEW, vol. 70, no. 9/10, September/October 1983, pages 318-323, Baden, CH; H. VOGT: "Linear accelerator dynaray-CH: a central component of the BBC radiotherapy system" * Page 320; page 323, right-hand column; figure 4 * | 1,6,10 | **TECHNICAL FIELDS SEARCHED (Int. Cl.4)** A 61 N |
| A | FR-A-2 466 084 (CGR) * Page 4, lines 16-27; figures 3,4 * | 1,2,10 | |

--- -/-

The present search report has been drawn up for all claims

| Place of search | Date of completion of the search | Examiner |
|---|---|---|
| THE HAGUE | 07-09-1987 | SCHMIERER U.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | MEDICAL & BIOLOGICAL ENG. & COMPUTING, vol. 19, no. 5, September 1981, pages 612-616, IFMBE, Stevenage, Herts, GB; J.A. BRACE et al.: "Computer-controlled cobalt unit for radiotherapy" * Pages 612-615 * | 1,2,5-7,10 | |
| | --- | | |
| A | IEEE TRANSACTIONS ON NUCLEAR SCIENCE, vol. NS-26, no. 2, April 1979, pages 2277-2280, IEEE, New York, US; L. BESSE et al.: "Therapy controller designed for patient irradiation at the SIN pi-applicator" | | |
| | ----- | | |

TECHNICAL FIELDS
SEARCHED (Int. Cl.4)

The present search report has been drawn up for all claims

| Place of search | Date of completion of the search | Examiner |
|---|---|---|
| THE HAGUE | 07-09-1987 | SCHMIERER U.J. |